# FASCICULE DE BREVET EUROPEEN

(11) **EP 2 600 149 B1**
(45) Date de publication et mention de la délivrance du brevet: **18.04.2018**
(21) Numéro de dépôt: 12194980.4
(22) Date de dépôt: 30.11.2012
(51) Int. Cl.: G01N 33/543, G01N 33/50, G01N 21/552, B01L 3/00

(54) **Procédé de mesure en temps réel des sécrétions individuelles d'une cellule**
Echtzeitmessverfahren der individuellen Sekretionen einer Zelle
Method for real-time measurement of the individual secretions of a cell

(30) Priorité: 30.11.2011 FR 1160964
(43) Date de publication de la demande: 05.06.2013
(73) Titulaire: COMMISSARIAT À L'ÉNERGIE ATOMIQUE ET AUX ÉNERGIES ALTERNATIVES, 75015 Paris (FR); Centre National de la Recherche Scientifique (C.N.R.S.), 75794 Paris Cedex 16 (FR); Institut National de la Santé et de la Recherche Médicale (INSERM), 75013 Paris (FR)
(72) Inventeur: Livache, Thierry, 38560 Jarrie (FR); Roupioz, Yoann, 38570 Theys (FR); Milgram, Sarah, 38570 Moretel Des Mailles (FR); Maillere, Bernard, 78000 Versailles (FR); Marche, Patrice, 38240 Meylan (FR)
(74) Mandataire: Gevers & Orès

(56) Documents cités:
- WO-A1-2005/106482
- WO-A1-2010/065372
- US-A1- 2008 096 241
- MILGRAM S ET AL: "On chip real time monitoring of B-cells hybridoma secretion of immunoglobulin", BIOSENSORS AND BIOELECTRONICS, ELSEVIER BV, NL, vol. 26, no. 5, 15 janvier 2011 (2011-01-15), pages 2728-2732, XP027580230, ISSN: 0956-5663 [extrait le 2010-09-29]
- SHIRASAKI Y ET AL: "Single cell real time secretion assay using amorphous fluoropolymer microwell array", 2011 16TH INTERNATIONAL SOLID-STATE SENSORS, ACTUATORS AND MICROSYSTEMS CONFERENCE (TRANSDUCERS 2011) : BEIJING, CHINA, 5 - 9 JUNE 2011, IEEE, PISCATAWAY, NJ, 5 juin 2011 (2011-06-05), pages 755-758, XP031910671, DOI: 10.1109/TRANSDUCERS.2011.5969330 ISBN: 978-1-4577-0157-3
- ALEXANDER REVZIN ET AL: "Biosensors for immune cell analysis-A perspective", BIOMICROFLUIDICS, vol. 6, no. 2, 1 janvier 2012 (2012-01-01) , pages 021301-1-021301-1, XP055049362, ISSN: 1932-1058, DOI: 10.1063/1.4706845

## Description

### Domaine de l'invention

La présente invention concerne un procédé pour la détermination et la quantification de composés sécrétés par une cellule individuelle en temps réel.

### Arrière plan technique

La communication entre cellules est un événement biologique de première importance. Elle peut avoir lieu pour permettre un transfert de signal entre des êtres unicellulaires, mais aussi et surtout, entre les cellules d'un même organisme. Les modes de communication peuvent avoir différentes formes, comme un contact physique entre cellules ou bien l'émission de composés par une cellule et la réception de ces composés par une autre cellule. Dans ce dernier mode, qui est un mode de communication chimique, c'est un ensemble de paramètres, dont la nature, la quantité, la fréquence et la durée de sécrétion des composés, qui porte l'information, ainsi que le type cellulaire de la cellule émettrice et le type cellulaire de la cellule réceptrice (ou cellule "cible") du message.

Ainsi, le réseau complexe des activités sécrétrices est l'objet de nombreuses investigations biomédicales : il s'agit de l'ensemble des cascades immunitaires, dont d'une part, le fonctionnement normal est impliqué dans certaines maladies, par exemple les infections les cancers et dont, d'autre part, les dysfonctionnements peuvent se traduire dans des états pathologiques comme une maladie auto-immune ou une allergie. Par ailleurs, la manipulation des cascades immunitaires présente un intérêt pour prévenir certains états pathologiques comme c'est le cas pour les vaccins ou le traitement des rejets de greffe. La possibilité de suivre le déclenchement d'une réponse immunitaire est donc un enjeu important pour de nombreuses applications biomédicales diagnostiques et thérapeutiques.

Dans ce cadre, il est important de pouvoir détecter les sécrétions cellulaires à l'échelle d'une cellule unique afin de différencier les différentes populations cellulaires concernées.

A l'heure actuelle, un seul produit permettant l'analyse de la réponse immunitaire à l'échelle cellulaire est approuvé pour une utilisation diagnostique. Il s'agit du test de diagnostic de la tuberculose T-SPOT®.*TB* (*Oxford Immunotec*), notamment décrit dans la demande internationale WO 9823960 et dans l'article de Leung et al. (2010) American Journal of Respiratory and Critical Care Medicine 182:834-840. Ce test se base sur la technique dite ELISPOT mise au point dans les années 1980 (Czerkinsky et al. (1983) J. Immunol. Methods 65:109-21). Cette technique implique la culture, dans des plaques multipuits, d'échantillons cellulaires pendant des intervalles de temps plus ou moins longs (de quelques heures à quelques jours), suivie du lavage de ces puits (élimination des cellules sécrétrices), et d'une succession d'étapes de lavage/révélation selon le principe du test *sandwich* ELISA : le fond de ces puits étant recouvert d'anticorps spécifiques d'un composé sécrété, un montage moléculaire à l'aide d'un deuxième anticorps suivi d'une révélation colorimétrique permet de visualiser *a posteriori* des taches ou « *spots »* à l'emplacement où une cellule a sédimenté pendant la culture et a sécrété un composé dans le milieu de culture. La diffusion des composés dans l'environnement proche d'une cellule permet donc de faire apparaître des taches caractéristiques d'une activité sécrétrice cellulaire individuelle.

Toutefois, bien que de plus en plus utilisée, cette approche souffre de différentes limites, dont un temps d'incubation relativement long (de un à deux jours) avant l'étape de révélation, ainsi que l'impossibilité de collecter les échantillons contenant des cellules sécrétrices puisque celles-ci sont lysées avant de procéder à l'étape de marquage. Enfin, il faut préciser que la technique ELISPOT ne permet pas de donner accès aux paramètres cinétiques de sécrétion cellulaire.

Différentes techniques permettant d'avoir accès aux sécrétions cellulaires en temps réel sont connues.

Il est ainsi possible de citer le procédé de *patch-clamp* dans lequel il est possible de quantifier, par mesure de capacitance et/ou ampérométrique, la sécrétion de composés électriquement chargés par la partie de la membrane plasmique obstruant la pipette (voir par exemple Westerink & Ewing (2008) Acta Physiol. (Oxf) 192:273-85). Ce procédé présente toutefois plusieurs inconvénients. Il nécessite tout d'abord d'isoler une cellule. Ensuite, il ne permet pas d'avoir accès aux sécrétions d'une cellule dans son ensemble. Enfin, il ne permet que de mesurer la sécrétion de composés électriquement chargés, de surcroit de manière non spécifique.

D'autres techniques impliquent la révélation chimique ou enzymatique en temps réel de composés sécrétés. Il s'agit, dans ces techniques, d'ajouter dans le milieu de culture cellulaire un ou plusieurs réactif(s) susceptible(s) de réagir avec le produit sécrété par des cellules. Ce peut être par exemple du luminol réagissant avec les espèces réactives de l'oxygène qui sont produites par des monocytes lors de leur différentiation en macrophages (Kasai et al. (2005) Analytica Chimica Acta 549:14-19) ou des enzymes métabolisant les produits libérés (Clark et al. (2009) Analytical Chemistry 81:2350-2356 ; Inoue et al. (2010) Biosens Bioelectron. 25:1723-8). Cependant, en plus de nécessiter l'ajout d'éléments non indispensables à la culture cellulaire, ces approches se limitent à l'étude de composés sécrétés qui doivent, soit avoir des propriétés chimiques particulières, soit être des substrats enzymatiques (comme le glycérol par exemple) pour être détectés. De fait, cette technique exclut la quasi-totalité des protéines que l'on peut trouver dans un échantillon biologique, ce qui pourrait expliquer l'absence d'exemple d'application de cette technique à la détection de cytokines.

Enfin, il a été récemment montré que des techniques optiques, notamment l'imagerie de résonance plasmonique de surface (SPRi), pouvaient être mises en oeuvre avec succès pour la détection de sécrétions cellulaires, mais sans permettre de mesurer une sécrétion spécifique à l'échelle de cellules individuelles.

Ainsi, Stybayeva et al. (2010) Colloids Surf. B. Biointerfaces 80:251-5 décrivent une analyse à l'aide de deux modules, ou compartiments : l'un dédié à la capture spécifique de types cellulaires d'intérêt, l'autre à l'analyse du milieu extra-cellulaire conditionné. Toutefois, ce montage ne permet pas d'établir un lien entre un produit sécrété et une cellule sécrétrice particulière.

Par ailleurs, Peterson et al. (2009) BMC Cell. Biol. 10:16 décrivent une méthode mettant en évidence la sécrétion, à l'échelle cellulaire, de biomolécules dans le milieu extra-cellulaire, mais ne permettent pas d'identifier la nature des produits sécrétés.

Milgram et al. (2011) Biosens. Bioelectron. 26:2728-2732 et Milgram et al. (2009) IEEE Sensors Journal 1768-1771, décrivent une méthode permettant l'évaluation, en temps réel, de la présence d'un analyte dans une solution. L'avantage principal de la méthode Milgram *et al.* est la détection en temps réel de l'analyte en solution, ce qui n'est pas possible à l'aide de l'ELISA. Toutefois cette méthode ne donne pas accès aux sécrétions individuelles d'une cellule.

Enfin, Shirasaki Y. et al. "Single cell real time sécrétion assay using amorphous fluoropolymer microwell array", 16th International Solid-State Sensors, Actuators and Microsystems Conférence (Transducers'11): Beijing, Chine, 5-9 Juin 2011, IEEE, p.755-758 décrivent la mesure (quantitative) en temps réel par la technique d'imagerie TIRF (Total Internal Reflection Fluorescent Imaging) de la sécrétion d'IL-1β par des mastocytes au niveau individuel à l'aide d'un anticorps fixé sur un support et d'un anticorps de détection fluorescent.

### Rescription de l'invention

La présente invention découle de la mise en évidence inattendue, par les inventeurs, qu'il était possible de suivre, en temps réel et de façon spécifique, la sécrétion d'un composé par une cellule individuelle par imagerie de résonance plasmonique de surface.

Ainsi, la présente invention concerne un procédé de mesure en temps réel de la sécrétion d'au moins un composé par au moins une cellule individuelle, comprenant :
- la culture en milieu liquide ou fluide d'au moins une cellule dans une chambre de culture dont au moins une paroi comprend au moins une zone sensible, la paroi de la chambre de culture comprenant la zone sensible étant une paroi basale vers laquelle les cellules peuvent sédimenter, la quantité de cellules dans la chambre de culture rapportée à la surface de la zone sensible étant de 1 à 10⁶ cellules/mm², la zone sensible comprenant au moins un ligand fixé sur un support solide à une densité de 10⁸ à 10 ligands/mm², le ligand pouvant se lier spécifiquement au composé ; et un élément de mesure en temps réel d'un signal produit lors de la liaison du composé au ligand, ledit signal ne nécessitant pas la médiation de molécules présentes dans la chambre de culture ou ajoutées à la chambre de culture, autres que le composé et le ligand, pour être produit ;
- l'identification d'au moins une tache correspondant à une portion continue et discrète de la zone sensible émettant un signal produit par la liaison d'au moins un composé respectivement à au moins un ligand ;
- la mesure en temps réel du signal produit par la tache identifiée, représentant la quantité de composé sécrétée par une cellule individuelle,
ledit signal étant mesuré par résonance plasmonique de surface.

Comme on l'entend ici, le « signal » est produit lors et/ou à partir de la liaison du composé au ligand et est mesurable par l'élément de mesure du signal. La « mesure » d'un signal désigne la détermination de la présence ou de l'absence du signal, et/ou sa quantification. L'expression en « temps réel » signifie que le signal est produit et mesuré essentiellement au moment où la liaison du composé et du ligand a lieu.

La mesure du signal selon l'invention est effectuée directement. Ainsi, le signal ne nécessite pas la médiation de molécules présentes dans la chambre de culture ou ajoutées à la chambre de culture, autres que le composé et le ligand, pour être produit. A titre d'exemple, le signal selon l'invention ne provient pas d'une sonde d'oxydoréduction, ou de la liaison additionnelle d'un marqueur spécifique du composé, tel qu'un anticorps marqué, sur un composé déjà fixé par le ligand. La mesure du signal selon l'invention peut être effectuée par un élément tel qu'un système de lecture optique comme un imageur de résonance plasmonique de surface. Dans le cadre de l'invention, le signal est mesuré par une technique optique sensible à des interactions se déroulant sur un support, ici la résonance plasmonique de surface. Les systèmes de lecteur optique de résonance plasmonique de surface sont bien connus de l'homme du métier et combinent généralement une source lumineuse, par exemple de type LED, afin de provoquer une excitation plasmonique et une caméra, par exemple de type CCD, pour enregistrer le signal produit par la résonance plasmonique. A ce titre, on préfère que le signal soit suivi en mode imagerie, plutôt qu'en mode de lecture monopoint, en particulier afin de suivre en temps réel les perturbations liées à des évènements cellulaires. Comme l'homme du métier le sait bien, le mode imagerie consiste à suivre les variations de signal de tous les pixels constituant l'image de la caméra CCD utilisée, alors que le mode monopoint consiste lui à définir, ou à prédéfinir, des régions de l'image, c'est-à-dire un ensemble de pixels, dont la moyenne des signaux obtenus reflète un signal moyen de la région de l'image choisie.

Le signal est mesuré sur une « tache » c'est-à-dire une portion continue et discrète de la zone sensible. Il s'agit d'une portion de la zone sensible où plusieurs ligands localisés à proximité les uns des autres se sont liés chacun à un composé et, le cas échéant, qui est séparée d'une ou plusieurs autres portions semblables de la zone sensible. La tache peut également être nommée « *spot* », en référence à la technique ELISPOT, et correspond à une portion de la zone sensible en contact avec les sécrétions d'une cellule individuelle. Une portion est dite continue lorsqu'elle ne comprend pas plusieurs sous-portions distinctes émettant un signal, séparées par des sous-portions n'émettant pas de signal ; autrement dit l'ensemble de la portion continue émet un signal. Une portion est dite discrète lorsqu'elle est d'une surface limitée, en rapport avec distance de diffusion maximale des sécrétions provenant d'une cellule au cours de la culture. En conséquence, la mesure du signal émis par une tache, c'est à dire une portion continue et discrète, de la zone sensible vise à s'assurer que l'on mesure bien les sécrétions provenant d'une cellule individuelle.

Une chambre de culture selon l'invention peut comprendre une ou plusieurs zones sensibles, chaque zone sensible pouvant être spécifique d'un seul composé ou de composés différents. Comme cela apparaîtra clairement à l'homme du métier la spécificité de chaque zone sensible vis-à-vis d'un ou plusieurs composés et portée par le ou les ligands qui la composent. Ainsi, chaque zone sensible selon l'invention peut comprendre une pluralité de ligands, identiques ou différents, se liant spécifiquement à un même composé, ou plusieurs groupes de ligands respectivement spécifiques de plusieurs composés différents.

Par ailleurs, on peut envisager que le procédé selon l'invention mette en oeuvre, outre une zone sensible, une zone du support solide ne comprenant pas de ligand ou comprenant un ligand non-spécifique du composé, afin de pouvoir mesurer les signaux provenant d'une interaction non spécifique du composé avec le support solide ou avec un ligand.

Le procédé de l'invention permet de mesurer les sécrétions provenant d'une cellule individuelle, autrement dit une seule et même cellule, c'est à dire que les sécrétions provenant d'une cellule unique peuvent être mesurées et distinguées des sécrétions de cellules qui seraient également présentes dans la chambre de culture que ces cellules soient du même type cellulaire, cas d'une population homogène de cellules, ou de types cellulaires différents, cas d'une population hétérogène de cellules.

Comme cela apparaitra de manière claire à l'homme du métier, la cellule individuelle selon l'invention peut être de toute origine phylogénétique ou histologique. En particulier, la cellule individuelle selon l'invention peut provenir de cellules qui sont isolées dans leur environnement naturel ou bien de cellules qui sont associées à d'autres cellules, que ce soit par des interactions physiques directes, transitoires ou non, ou par des interactions à distance, notamment par l'émission et la réception de messagers biochimiques.

Ainsi, de préférence, la cellule selon l'invention est sélectionnée dans le groupe constitué de cellules bactériennes, de cellules de champignon, de cellules d'algues, de cellules de protozoaire, et de cellules de métazoaire, tel qu'un végétal ou un animal. Par ailleurs, différents types de cellules peuvent être présents dans la chambre de culture.

De préférence également, la cellule selon l'invention provient d'un échantillon biologique, d'un échantillon alimentaire, d'un échantillon d'eau, notamment d'eau douce, d'eau de mer ou d'eau usée, d'un échantillon de sol, d'un échantillon de boue et d'un échantillon d'air. Plus préférablement, la cellule selon l'invention provient d'un échantillon biologique, tel que :
- du sang,
- du liquide céphalorachidien,
- une sécrétion buccale, notamment de la salive,
- du sperme,
- une sécrétion vaginale,
- de l'urine,
- des selles,
- du liquide synovial,
- une biopsie,
- un prélèvement provenant d'un lavage d'organe ou de cavité anatomique, en particulier un lavage du nez, de la bouche, de la gorge, de l'estomac, des intestins, du vagin, ou un lavage bronchoalvéolaire des poumons,
- un échantillon de drainage d'un liquide biologique, en particulier du liquide d'ascite, du liquide lymphatique, du pus, ou de la bile ou
- un prélèvement d'une sérosité cutanée ou conjonctivale.

On préfère aussi que la cellule selon l'invention soit sélectionnée dans le groupe constitué de cellules immunitaires, de cellules nerveuses, de cellules endocrines, de cellules souches, de cellules épithéliales, de cellules infectées par un agent infectieux, tel qu'un virus, une bactérie ou un protozoaire, et de cellules de cancer.

Le composé selon l'invention peut être de tout type susceptible d'être secrété par une cellule.

Ainsi, sur un plan structural, il peut s'agir de composés unimoléculaires ou pluri-moléculaires résultant de l'association non covalente d'un nombre limité de sous-unités, généralement moins de 10 sous-unités, tels qu'une protéine, un polypeptide, ou un peptide, un lipide, une glycoprotéine, un glycolipide, une lipoprotéine, un ion inorganique, ou une petite molécule organique, comprenant de préférence de 1 à 100 atomes de carbone. Il peut également s'agir de composés particulaires ou supramoléculaires, tels qu'une vésicule, notamment un exosome ou une particule microbienne, telle qu'une particule virale par exemple.

Par ailleurs, sur un plan fonctionnel, le composé selon l'invention est de préférence sélectionné dans le groupe constitué :
- de composés de signalisation intercellulaire, tels qu'une hormone, un neurotransmetteur une cytokine ou une chimiokine,
- d'une protéine de la matrice extra-cellulaire ;
- d'une immunoglobuline ;
- d'un agent infectieux, tel qu'une bactérie, un virus, ou un parasite protozoaire, ou d'une sous-unité d'un agent infectieux, c'est-à-dire un constituant ou une production d'un agent infectieux, tel qu'une protéine ou une particule virale ou une toxine bactérienne.

Le ligand selon l'invention est spécifique du composé, c'est à dire qu'il se lie spécifiquement ou préférentiellement au composé lorsque le composé est présent dans un mélange comprenant des molécules différentes. Le ligand selon l'invention peut être de toute nature. Ainsi il peut s'agir d'un peptide, d'un polypeptide, d'une protéine, d'une glycoprotéine, d'un oligosaccharide, d'un polysaccharide, d'un acide nucléique, d'un lipide, d'un glycolipide, d'une lipoprotéine, d'un polymère, notamment adapté à la fixation spécifique d'ions inorganiques, tel que ceux décrits par Lange et al. (2008) Anal. Chim. Acta 614:1-26, ou à la fixation d'un sucre, tel que le glucose, en particulier par un polymère du type poly(N-isopropylacrylamide) (pNIPAM) ou pNIPAM-co-acrylamidophénylboronate (pNIPAM-co-APBA), ou d'un composé organique comprenant de 1 à 100 atomes carbone. De préférence, le ligand selon l'invention est sélectionné dans le groupe constitué d'un anticorps, d'un fragment d'anticorps, notamment comprenant la partie de liaison à l'antigène de l'anticorps, d'un scFv, d'un antigène, d'un haptène, d'un aptamère, d'une lectine, et d'un agent chélatant.

Le support solide est adapté à la mesure du signal. Il peut s'agir d'un support en verre ; en silicium ; en matériau polymère organique, en particulier de type acrylate, polydiméthylsiloxane (PDMS), copolymère oléfinique cyclique (COC), polyéther éther cétone (PEEK), nitro-cellulose ; en matériau métallique, en particulier en argent, en or, ou en platine ; d'un conducteur carboné, en particulier de type carbone vitreux, graphite, graphène, nanostructure carbonée, ou diamant ; ou d'une combinaison d'au moins deux de ces matériaux. Comme la mesure est effectuée par résonance plasmonique de surface, le support solide est approprié à une excitation plasmonique et à la propagation d'une onde évanescente à sa surface. On préférera un matériau transparent, par exemple du verre, recouvert d'une couche métallique, notamment d'or, en particulier d'épaisseur 10 à 100 nm, et plus particulièrement d'épaisseur 50 nm.

La fixation du ligand sur le support solide pour former la zone sensible selon l'invention peut être effectuée par toute technique adaptée. On peut en particulier envisager une simple adsorption, des interactions électrostatiques, ou un greffage covalent. A titre d'exemple, lorsque le support solide est du verre, on préférera utiliser des réactifs de type silane ; lorsque le support solide est en or ou comprend une partie en or, on préférera complexer des ligands sur le métal par l'intermédiaire d'une fonction thiol. Il est également possible d'immobiliser les ligands dans des matrices polymères. La densité de ligand dans une zone sensible selon l'invention est de 10⁸ à 10¹² ligands/mm².

La culture selon l'invention est conduite *in vitro* dans un milieu permettant la diffusion du composé depuis les cellules vers les ligands. En outre, comme cela apparaîtra de manière claire à l'homme du métier, la culture selon l'invention est conduite de manière à obtenir la survie des cellules et à permettre la sécrétion du composé par les cellules. Ainsi, la culture selon l'invention est conduite en milieu liquide ou fluide, tel qu'un milieu gélifié. Les techniques et les conditions de culture de cellules, notamment en milieu liquide ou fluide, sont bien connues de l'homme du métier, qui sait notamment définir, pour chaque microorganisme, et chaque type cellulaire, le milieu nutritif adéquat, la température de culture optimale, par exemple 37°C pour de nombreuses cellules de mammifères, ainsi que l'atmosphère nécessaire au maintient de la viabilité des cellules. La durée de culture est également adaptable pour chaque type cellulaire, en fonction de sa vitesse de sécrétion. Généralement, la durée de culture sera inférieure à 24 h. En outre, la culture pourra être stoppée dès que l'information recherchée, telle que la détection d'un composé secrété, aura pu être obtenue.

Comme l'homme du métier le comprendra bien, lorsque la cellule selon l'invention provient d'un échantillon ou d'une composition, la culture selon l'invention de la cellule peut être mise en oeuvre directement à partir de l'échantillon ou de la composition, ou bien après isolement et/ou concentration des cellules. La paroi de la chambre de culture comprenant au moins une zone sensible selon l'invention est une paroi basale vers laquelle les cellules peuvent sédimenter. La quantité de cellules dans la chambre de culture rapportée à la surface de la zone sensible est de 1 à 10⁶ cellules/mm².

Par ailleurs, on préfère que le procédé mette en oeuvre la culture d'une suspension de cellules, notamment une suspension homogène de cellules, c'est-à-dire une suspension dans laquelle les cellules sont du même type, dont la concentration est inférieure à 200 000 cellules par millilitre, et plus préférentiellement dont la concentration est comprise entre 1000 et 50 000 cellules par millilitre. A titre d'exemple, dans le cas d'une population de cellules issues d'un même hydridome secrétant un anticorps monoclonal, on cultivera une suspension de cellules comprenant de 10 000 à 50 000 cellules par millilitre.

Dans le cas, où la suspension n'est pas homogène, c'est-à-dire qu'il s'agit d'une suspension complexe comprenant plusieurs types et/ou sous-types de cellules différents et dont seulement une fraction des cellules sécrète des composés selon l'invention, on préfère alors que la fraction de cellules sécrétrices soit inférieure à 90 % du nombre total de cellules présentes dans la suspension. En particulier, on préfère que la fraction de cellules sécrétrice représente entre 0,0001 % et 50 % du nombre total de cellules présentes dans la suspension. A titre d'exemple, dans le cas d'une suspension cellulaire non homogène constituée de splénocytes, on peut, selon l'invention, mesurer la sécrétion d'interféron gamma (IFNγ) par au moins un lymphocyte T individuel, les lymphocytes T représentant 35 à 40 % de la population totale de splénocytes de la suspension. Dans un autre exemple, dans le cas d'une suspension cellulaire non homogène de lymphocytes T, on peut, selon l'invention, mesurer la sécrétion d'interleukine 2 (IL2) par au moins un lymphocyte T CD4+ individuel, les lymphocytes T CD4+ représentant environ 60 % de la population totale lymphocytes T de la suspension.

### Description des figures

Figure 1

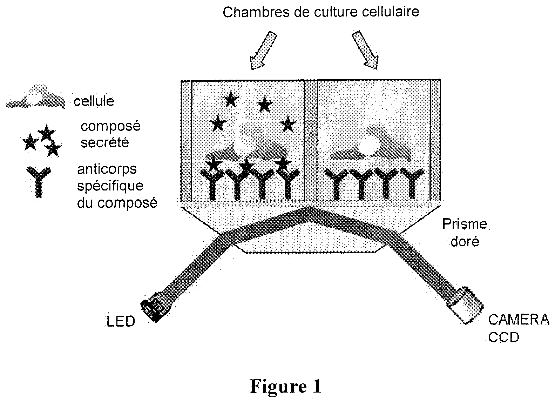

   La figure 1 est une représentation schématique du dispositif expérimental mis en oeuvre dans l'Exemple. La zone sensible du dispositif (prisme doré) est chimiquement modifiée par la fixation d'anticorps spécifiques d'un composé sécrété par des cellules. Deux échantillons cellulaires ont été cultivés conjointement sur le dispositif : dans la chambre de culture de gauche, des cellules sécrétant le composé d'intérêt et dans la chambre de culture de droite des cellules ne sécrétant pas cette molécule. Cette configuration à deux chambres de culture permet de mesurer et quantifier les signaux parasites, non spécifiques. Le composé sécrété est détecté en temps réel et sans marquage par SPRi à l'aide d'une LED, d'un filtre polariseur et d'une caméra CCD.
Figure 2

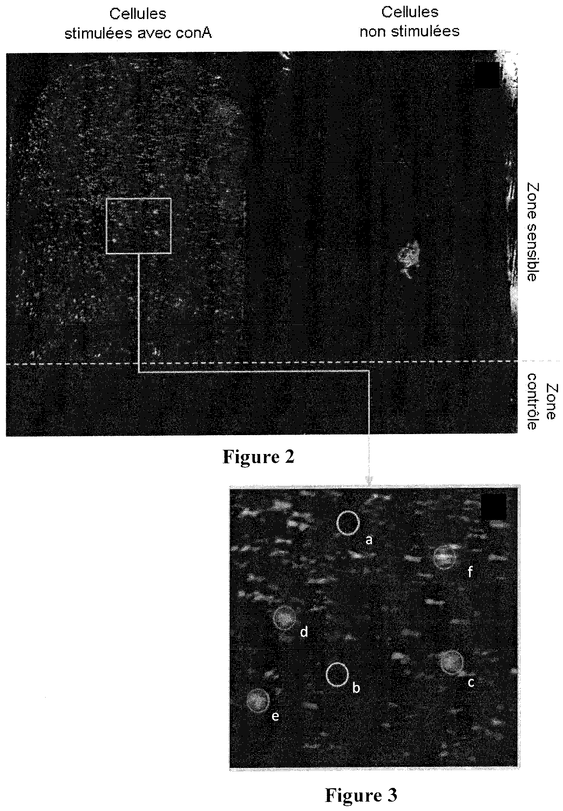

   La figure 2 représente une image SPR de la surface du prisme doré du dispositif utilisé dans l'Exemple. Cette surface est divisée en une zone sensible (en haut) fonctionnalisée avec l'anticorps et une zone contrôle (en bas) non fonctionnalisée. Sur ces zones, ont été incubées les cellules dans 2 chambres de culture distinctes, l'une dans laquelle les cellules ne sont pas stimulées (à droite), l'autre dans lequel les cellules sont stimulées avec de la ConA (à gauche). Des taches (ou *« spots* ») claires correspondant aux composés sécrétés fixés sur la surface sensible sont apparus sur la partie de la zone sensible sur laquelle ont été incubées les cellules stimulées par la ConA (en haut à gauche). La partie de la zone sensible encadrée en blanc est agrandie dans la Figure 3.
Figure 3
   La figure 3 est un agrandissement de la partie de la zone sensible encadrée en blanc dans la figure 2. Des masques en forme de cercle sont représentés, ils délimitent des portions d'intérêt de la zone sensible correspondant à une tache (ou *« spot* ») (cercles c, d, e, et f). Des portions témoins (sans tache) ont également été sélectionnées (cercles a et b).
Figure 4

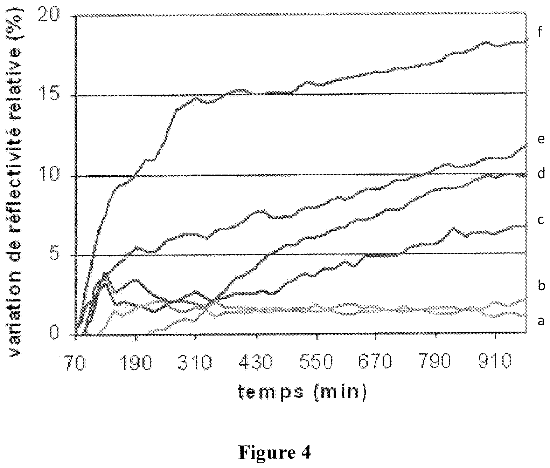

   La figure 4 représente la variation de réflectivité relative (axe des ordonnées, en %) des portions a, b, c, d, e, et f de la zone sensible représentés dans la figure 3 en fonction du temps (axe des abscisses, en minutes).

### EXEMPLE

Dans cet exemple, l'interféron gamma (IFNγ) sécrété par des splénocytes de souris a été détecté en temps réel, à l'échelle de cellules individuelles par imagerie de résonance plasmonique de surface (SPRi), suite à une stimulation cellulaire utilisant la Concanavaline A (conA). Les cellules stimulées ont été étudiées, ainsi que des cellules non stimulées, donc ne sécrétant pas l'IFNγ, utilisées comme contrôle négatif sur le même dispositif.

### 1. Dispositif expérimental et réactifs

Dans cet exemple, le dispositif (voir la **Figure 1**) comprend :
- deux chambres de culture et d'analyse contenant du milieu AIMV (Gibco) permettant le maintien en culture (viabilité et activité sécrétrice) de deux échantillons cellulaires en parallèle ;
- un anticorps monoclonal de rat dirigé contre l'interféron gamma de souris (anti-IFNγ, BD Biosciences) utilisé en tant que ligand spécifique pour le produit sécrété ;
- un prisme en verre recouvert d'un film de 50 nm d'or (GenOptics) en tant que support solide de l'anticorps anti-IFNγ ;
- un imageur par résonance plasmonique de surface SPRi-lab+ (GenOptics) en tant qu'élément de transduction du signal permettant une lecture optique en temps réel des interactions entre l'anticorps (IgG anti-INFγ) et le composé sécrété (INFγ de souris) ;
- une étuve (Memmert) permettant de maintenir l'ensemble du dispositif à 37°C, température favorable à la sécrétion cellulaire.

Réactifs nécessaires :
- Tampon phosphate salin (PBS, Sigma-Aldrich) ;
- Milieu de culture Roswell Park Mémorial Institute (RPMI, Sigma-Aldrich)
- Acide 11-mercapto-undécanoïque (Sigma-Aldrich) ;
- N,N'-Dicyclohexylcarbodiimide (DCC, Sigma-Aldrich) ;
- N-hydroxysuccinimide (NHS, Sigma-Aldrich) ;
- diméthylformamide (DMF, Sigma-Aldrich) ;
- Solution antibiotique: pénicilline 5000U/ml - streptomycine 5mg/ml (Sigma-Aldrich) ;
- Concanavaline A (Sigma-Aldrich).

### 2. Fabrication de la zone sensible : fixation des anticorps sur la surface d'or

Les anticorps ont été fixés sur la surface d'or par complexation de produits thiolés. Brièvement, les anticorps ont été couplés à une molécule de 11-mercapto-undecanoyl-1-N-hydroxysuccinimide ester (Thiol-NHS), synthétisée à partir d'acide 11-mercapto-undecanoïque, N,N'-Dicyclohexylcarbodiimide (DCC) et N-hydroxysuccinimide (NHS) dans du diméthylformamide (DMF). Le couplage avec l'anticorps a été effectué dans du PBS équilibré à pH8, une nuit à 4°C, avec un ratio molaire de Thiol-NHS/anticorps de 10:1. Les anticorps ainsi couplés ont ensuite été purifiés par ultracentrifugation sur une membrane présentant un seuil de coupure à 30 kDa, puis concentrés à 2µM dans du PBS contenant 10% de glycérol. Les anticorps couplés ont été déposés et étalés sur la surface d'or et ainsi greffés par liaison Soufre-Or. Une zone contrôle non fonctionnalisée et une zone sensible, fonctionnalisée avec les anticorps, ont été délimitées sur la même puce.

### 3. Culture cellulaire

Les splénocytes murins ont été prélevés chez une souris de type C57B1/6. Après prélèvement de la rate, les cellules ont été dissociées sur une grille en tamis et suspendues dans du milieu RPMI. Après centrifugation de 5 min à 300g, la suspension cellulaire a été incubée pendant 5 min en présence d'un tampon de lyse des globules rouges (8,3 g/l de NH₄Cl, 0,8 g/l de NaHCO₃, 0,04g/l d'EDTA) afin d'éliminer ce type cellulaire de l'échantillon. Après un lavage dans du PBS, les cellules ont été à nouveau centrifugées (5 min, 300g) et mises en suspension à une densité de 10⁶ cellules/ml dans du milieu RPMI contenant 10% de sérum de veau foetal (SVF), 1% de solution antibiotique. La viabilité des cellules a été vérifiée par comptage après coloration au bleu trypan. Juste avant l'expérience, les cellules ont été centrifugées 5 min à 300 g et remises en suspension à une densité de 200 000 cellules/ml dans du milieu AIMV tamponné contenant ou non de la Concanavaline A à 2 µg/ml.

### 4. Détection de sécrétions cellulaires

Avant l'analyse, la zone sensible a été traitée par incubation dans du milieu AIMV pendant 30 min. Le prisme a ensuite été mis en place dans l'imageur SPR, sous les chambres de culture afin de compléter le dispositif. L'ensemble a été placé dans une étuve à 37°C. 500 µl de suspension cellulaire préparée comme décrit ci-dessus ont été déposés dans chacune des chambres de culture et incubé le temps de l'expérience. Ainsi, des cellules stimulées par la ConA ont été cultivées dans une chambre tandis que des cellules témoins non stimulées ont été incubées dans l'autre chambre. La liaison des composés sécrétés par les anticorps a été détectée par SPRi. Une caméra CCD 12-bit a permis la quantification du signal constitué des variations de réflectivité provoquées par l'interaction IFNγ-anticorps dans la zone sensible. Des images des variations de réflectivité à la surface de la zone sensible ont été enregistrées en temps réel.

Des taches ou « *spots* » sont apparues sur la surface de la zone sensible (**Figure 2**), formées par les composés sécrétées par chaque cellule individuelle liés par les anticorps de la zone sensible. Des « masques » ont alors été tracés sur les images (représentés par les cercles notés a, b, c, d, e, et f dans la **Figure 3**) afin de délimiter des portions de la zone sensible correspondant *a priori* à des anticorps liant l'IFNγ produit par une seule et même cellule (cercles c, d, e, et f). Des portions sans tache ont également été sélectionnées comme témoins (cercles a et b) dans la zone sensible en présence de cellules stimulées.

La moyenne de la variation de réflectivité par pixel contenus dans chacune des portions sélectionnées a été tracée au cours du temps pour chaque portion c, d, e, et f (de même pour les portions témoins a et b). La variation de "réflectivité relative" exprimée en pourcentage de réflectivité, des portions de la zone sensible contenant les cellules stimulées a été calculée par soustraction du bruit, c'est-à-dire de la variation de réflectivité moyenne enregistrée dans la partie de la zone sensible contenant les cellules témoins non-stimulées et donc non-sécrétrices (**Figure 4**).

Les courbes c, d, e, et f de la **Figure 4** montrent des variations de réflectivité avec des cinétiques propres à chaque tache, mettant ainsi en évidence des comportements individuels propre à chaque cellule.

## Revendications

1. Procédé de mesure en temps réel de la sécrétion d'au moins un composé par une cellule individuelle, comprenant :
- la culture en milieu liquide ou fluide d'au moins une cellule dans une chambre de culture dont au moins une paroi comprend au moins une zone sensible,
la paroi de la chambre de culture comprenant la zone sensible étant une paroi basale vers laquelle les cellules peuvent sédimenter,
la quantité de cellules dans la chambre de culture rapportée à la surface de la zone sensible étant de 1 à 10⁶ cellules/mm², la zone sensible comprenant au moins un ligand fixé sur un support solide à une densité de 10⁸ à 10¹² ligands/mm²,
le ligand pouvant se lier spécifiquement au composé,
et un élément de mesure en temps réel d'un signal produit lors de la liaison du composé au ligand, ledit signal ne nécessitant pas la médiation de molécules présentes dans la chambre de culture ou ajoutées à la chambre de culture, autres que le composé et le ligand, pour être produit ;
- l'identification d'au moins une tache correspondant à une portion continue et discrète de la zone sensible émettant un signal produit par la liaison d'au moins un composé respectivement à au moins un ligand ;
- la mesure en temps réel du signal produit par la tache identifiée, représentant la quantité de composé sécrétée par une cellule individuelle,
ledit signal étant mesuré par résonance plasmonique de surface.

2. Procédé selon la revendication 1, dans lequel la cellule est sélectionnée dans le groupe constitué de cellules bactériennes, de cellules de champignon, de cellules d'algues, de cellules de protozoaire, et de cellules de métazoaire.

3. Procédé selon l'une des revendications 1 à 2, dans lequel la cellule provient d'un échantillon sélectionné dans le groupe constitué d'un échantillon biologique, d'un échantillon alimentaire, d'un échantillon d'eau, d'un échantillon de sol, d'un échantillon de boue et d'un échantillon d'air.

4. Procédé selon l'une des revendications 1 à 3, dans lequel la cellule provient d'un échantillon biologique sélectionné dans le groupe constitué du sang, du liquide céphalorachidien, d'une sécrétion buccale, du sperme, d'une sécrétion vaginale, de l'urine, des selles, du liquide synovial, d'une biopsie, d'un prélèvement provenant d'un lavage d'organe ou de cavité anatomique, d'un échantillon de drainage d'un liquide biologique, et d'un prélèvement d'une sérosité cutanée ou conjonctivale.

5. Procédé selon l'une des revendications 1 à 4, dans lequel la cellule est sélectionnée dans le groupe constitué de cellules immunitaires, de cellules nerveuses, de cellules endocrines, de cellules souches, de cellules épithéliales, de cellules infectées par un agent infectieux, et de cellules de cancer.

6. Procédé selon l'une des revendications 1 à 5, dans lequel le composé est sélectionné dans le groupe constitué d'une protéine, d'un polypeptide, d'un peptide, d'un lipide, d'une glycoprotéine, un glycolipide, une lipoprotéine, un ion inorganique, une petite molécule organique, une vésicule, une particule microbienne et une particule virale.

7. Procédé selon l'une des revendications 1 à 6, dans lequel le composé est sélectionné dans le groupe constitué de composés de signalisation intercellulaire, d'une protéine de la matrice extra-cellulaire, d'une immunoglobuline, et d'un agent infectieux ou d'une sous-unité d'un agent infectieux.

8. Procédé selon l'une des revendications 1 à 7, dans lequel le ligand est sélectionné dans le groupe constitué d'un peptide, d'un polypeptide, d'une protéine, d'une glycoprotéine, d'un oligosaccharide, d'un polysaccharide, d'un acide nucléique, d'un lipide, d'un polymère, et d'un composé organique comprenant de 1 à 100 atomes carbone.

9. Procédé selon l'une des revendications 1 à 8, dans lequel le ligand est sélectionné dans le groupe constitué d'un anticorps, d'un fragment d'anticorps, d'un scFv, d'un antigène, d'un haptène, d'un aptamère, d'une lectine, et d'un agent chélatant.

10. Procédé selon l'une des revendications 1 à 9, dans lequel le support solide est constitué d'un matériau sélectionné dans le groupe constitué de verre, de silicium, d'un polymère organique, d'un matériau métallique et d'un conducteur carboné.

## Patentansprüche

1. Echtzeit-Messverfahren der Sekretion mindestens einer Verbindung durch eine individuelle Zelle, umfassend:
- das Kultivieren in flüssigem oder fluidem Medium mindestens einer Zelle in einer Kulturkammer, von der mindestens eine Wand mindestens eine sensible Zone umfasst,
wobei die Wand der Kulturkammer, die die sensible Zone umfasst, eine Basalwand ist, zu der die Zellen sedimentieren können,
wobei die Anzahl der Zellen in der Kulturkammer, die an der Oberfläche der sensiblen Zone angebracht ist, 1 bis 10⁶ Zellen/mm² beträgt,
wobei die sensible Zone mindestens einen Liganden umfasst, fixiert auf einem festen Träger, in einer Dichte von 10⁸ bis 10¹² Liganden/mm²,
wobei sich der Ligand spezifisch mit der Verbindung verbinden kann,
und ein Echtzeit-Messelement eines Signals, produziert bei der Verbindung der Verbindung mit dem Liganden, wobei das Signal nicht die Mediation von Molekülen benötigt, die in der Kulturkammer vorhanden sind oder der Kulturkammer hinzugefügt wurden, die andere als die Verbindung und der Ligand sind, um produziert zu werden,
- das Identifizieren mindestens eines Flecks, der einem kontinuierlichen und diskreten Abschnitt der sensiblen Zone entspricht, der ein Signal sendet, produziert durch die Verbindung mindestens einer Verbindung jeweils mit mindestens einem Liganden,
- das Messen in Echtzeit des von dem identifizierten Fleck produzierten Signals, das die Menge Verbindung darstellt, die von einer individuellen Zelle sekretiert wurde, wobei das Signal durch Oberflächenplasmonresonanz gemessen wird.

2. Verfahren nach Anspruch 1, wobei die Zelle aus der Gruppe ausgewählt ist, die von Bakterienzellen, Pilzzellen, Algenzellen, Einzellerzellen und Vielzellerzellen gebildet ist.

3. Verfahren nach einem der Ansprüche 1 bis 2, wobei die Zelle von einer Probe stammt, ausgewählt aus der Gruppe, die von einer biologischen Probe, einer Lebensmittelprobe, einer Wasserprobe, einer Bodenprobe, einer Schlammprobe und einer Luftprobe gebildet ist.

4. Verfahren nach einem der Ansprüche 1 bis 3, wobei die Zelle von einer biologischen Probe stammt, ausgewählt aus der Gruppe, die vom Blut, von der Rückenmarksflüssigkeit, von einer Mundsekretion, vom Sperma, von einer vaginalen Sekretion, vom Urin, vom Stuhl, von der Synovialflüssigkeit, von einer Biopsie, von einer Entnahme, die von einer Waschung eines Organs oder einer anatomische Kavität, von einer Drainageprobe einer biologischen Flüssigkeit und einer serösen Haut- oder Bindehautentnahme gebildet ist.

5. Verfahren nach einem der Ansprüche 1 bis 4, wobei die Zelle aus der Gruppe ausgewählt ist, die von Immunzellen, Nervenzellen, endokrinen Zellen, Stammzellen, Epithelialzellen, von einem infektiösen Mittel infizierten Zellen und Krebszellen gebildet ist.

6. Verfahren nach einem der Ansprüche 1 bis 5, wobei die Verbindung aus der Gruppe ausgewählt ist, die von einem Protein, einem Polypeptid, einem Peptid, einem Lipid, einem Glycoprotein, einem Glycolipid, einem Lipoprotein, einem anorganischen Ion, einem kleinen organischen Molekül, einem Mikrobenpartikel und einem viralen Partikel gebildet ist.

7. Verfahren nach einem der Ansprüche 1 bis 6, wobei die Verbindung aus der Gruppe ausgewählt ist, die von den interzellulären Signalverbindungen, einem Protein der extrazellulären Matrix, einem Immunglobulin und einem infektiösen Mittel oder einer Untereinheit eines infektiösen Mittels gebildet ist.

8. Verfahren nach einem der Ansprüche 1 bis 7, wobei der Ligand aus der Gruppe ausgewählt ist, die von einem Peptid, einem Polypeptid, einem Protein, einem Glycoprotein, einem Oligosaccharid, einem Polysaccharid, einer Nukleinsäure, einem Lipid, einem Polymer und einer organischen Verbindung, die 1 bis 100 Kohlenstoffatome umfasst, gebildet ist.

9. Verfahren nach einem der Ansprüche 1 bis 8, wobei der Ligand aus der Gruppe ausgewählt ist, die von einem Antikörper, einem Antikörperfragment, einem scFv, einem Antigen, einem Hapten, einem Aptamer, einem Lectin und einem chelatisierenden Mittel gebildet ist.

10. Verfahren nach einem der Ansprüche 1 bis 9, wobei der feste Träger von einem Material gebildet ist, das aus der Gruppe ausgewählt ist, die von Glas, Silizium, einem organischen Polymer, einem metallischen Material und einem kohlenstoffhaltigen Leiter gebildet ist.

## Claims

1. Method for real-time measurement of the secretion of at least one compound by an individual cell, comprising:
- culture, in a liquid or fluid medium, of at least one cell in a culture chamber having at least one wall that comprises at least one sensitive region,
the wall of the culture chamber comprising the sensitive region being a basal wall towards which the cells are able to sediment;
the ratio of the quantity of cells in the culture chamber to the surface area of the sensitive region being from 1 to 10⁶ cells/mm²;
the sensitive region comprising at least one ligand attached to a solid substrate at a density of 10⁸ to 10¹² ligands/mm²;
the ligand being able to bind specifically to the compound;
and an element for the real-time measurement of a signal produced at the time of binding of the compound to the ligand, said signal not requiring the mediation of molecules contained in the culture chamber or added to the culture chamber, other than the compound and the ligand, to be produced;
- identification of at least one spot corresponding to a continuous discrete portion of the sensitive region emitting a signal produced by the binding of at least one compound respectively to at least one ligand;
- real-time measurement of the signal produced by the identified spot representing the amount of compound secreted by an individual cell,
said signal being measured by surface plasmon resonance.

2. The method according to claim 1, wherein the cell is selected from the group composed of bacterial cells, fungus cells, algae cells, protozoan cells and metazoan cells.

3. The method according to one of claims 1 to 2, wherein the cell is derived from a sample selected from the group composed of a biological sample, a food sample, a water sample, a soil sample, a mud sample and an air sample.

4. The method according to one of claims 1 to 3, wherein the cell is derived from a biological sample selected from the group composed of blood, cerebrospinal fluid, buccal secretion, sperm, vaginal secretion, urine, stools, synovial fluid, a biopsy, a sample from washing of a body organ or anatomic cavity, sample from drainage of a biologic fluid, and a sample of cutaneous or conjunctival serous fluid.

5. The method according to one of claims 1 to 4, wherein the cell is selected from the group composed of immune cells, nerve cells, endocrine cells, stem cells, epithelial cells, cells infected with an infectious agent, and cancer cells.

6. The method according to one of claims 1 to 5, wherein the compound is selected from the group composed of a protein, polypeptide, peptide, lipid, glycoprotein, glycolipid, lipoprotein, inorganic ion, small organic molecule, a vesicle, a microbial particle and a viral particle.

7. The method according to one of claims 1 to 6, wherein the compound is selected from the group composed of intercellular signalling compounds, a protein of the extracellular matrix, an immunoglobulin, and an infectious agent or a subunit of an infectious agent.

8. The method according to one of claims 1 to 7, wherein the ligand is selected from the group composed of a peptide, polypeptide, protein, glycoprotein, oligosaccharide, polysaccharide, nucleic acid, lipid, polymer and an organic compound having 1 to 100 carbon atoms.

9. The method according to one of claims 1 to 8, wherein the ligand is selected from the group composed of an antibody, an antibody fragment, an scFv, an antigen, a hapten, an aptamer, a lectin and a chelating agent.

10. The method according to one of claims 1 to 9, wherein the solid support is composed of a material selected from the group composed of glass, silicon, an organic polymer, a metal material and a carbon conductor.
